# EUROPÄISCHE PATENTANMELDUNG

(11) **EP 4 501 955 A1**
(43) Veröffentlichungstag der Anmeldung: **05.02.2025**
(21) Anmeldenummer: 23189741.4
(22) Anmeldetag: 04.08.2023
(51) Int. Cl.: C07K 14/78, A61L 27/24, B29C 64/00, B33Y 10/00

(54) **POLYPEPTID FÜR EINE TINTE FÜR DEN 3D-DRUCK**

(71) Anmelder: Cellbricks GmbH, 13353 Berlin (DE)
(72) Erfinder: Schlauch, Domenic, 88131 Bodolz (DE); Ebbecke, Jan Peter, 37620 Halle (DE); Pepelanova, Iliyana, 31582 Nienburg (Weser) (DE); Pirmahboub, Hamidreza, 30161 Hannover (DE); von Arnim, Joachim, 10719 Berlin (DE); Kloke, Lutz, 12435 Berlin (DE)
(74) Vertreter: Eder Schieschke & Partner mbB

(57) **Zusammenfassung**

Die Erfindung betrifft ein Polypeptid, das sich hervorragend für die Anwendung in einer Tinte für den 3D-Druck eignet. Weiterhin betrifft die vorliegende Erfindung auch ein Polynucleotid, das das erfindungsgemäße Polypeptid codiert, sowie eine Wirtszelle, die das erfindungsgemäße Polypeptid exprimiert. Eine weitere Ausgestaltung ist ein Verfahren zur Herstellung einer Tinte für den 3D-Druck, wobei die Tinte ein Polypeptid enthält. Weitere Ausgestaltungen betreffen Verfahren zur Herstellung eines 3D-Gerüsts sowie das nach diesen Verfahren erhältliche 3D-Gerüst, unter anderem auch zur Verwendung in der Medizin.

## Beschreibung

Die vorliegende Erfindung betrifft ein Polypeptid gemäß dem Patentanspruch 1 oder 5, das sich hervorragend in einer Tinte für den 3D-Druck eignet. Weiterhin betrifft die vorliegende Erfindung auch ein Polynucleotid nach Patentanspruch 7, das das erfindungsgemäße Polypeptid codiert, sowie eine Wirtszelle nach Patentanspruch 8, die das erfindungsgemäße Polypeptid exprimiert. Eine weitere Ausgestaltung ist ein Verfahren nach Patentanspruch 9 zur Herstellung einer Tinte für den 3D-Druck, wobei die Tinte ein Polypeptid enthält. Weitere Ausgestaltungen betreffen Verfahren zur Herstellung eines 3D-Gerüsts nach den Patentansprüchen 11 und 13 sowie das nach diesen Verfahren erhältliche 3D-Gerüst nach den Patentansprüchen 15 und 16, unter anderem auch zur Verwendung in der Medizin.

In den letzten Jahren hat der 3D-Druck von 3D-Gerüsten mit einer Matrix aus Biopolymeren stark zugenommen. Diese 3D-Gerüste werden entweder zur nachträglichen Besiedelung von lebenden Zellen verwendet, oder die lebenden Zellen werden direkt bei dem Aufbau des 3D-Gerüsts in die Matrix aus dem Biopolymer mit eingebaut. Neben weiteren Anwendungsmöglichkeiten sollen hier auch lebende Gewebe und Organe, wie beispielsweise Brustimplantate aus lebenden Zellen, hergestellt werden, die in ein lebendes Wesen eingepflanzt werden können.

Dabei eignet sich allerdings nicht jegliches Biopolymer, da manche dieser Biopolymere Gruppen bzw. Abfolgen von Aminosäuren aufweisen, die eine Immunreaktion hervorrufen können, da das Immunsystem sie als Fremdproteine erkennt. Weiterhin müssen die Biopolymere bei einer physiologisch verträglichen Temperatur zu einem 3D-Gerüst verarbeitbar sein, d.h. in der Tinte gelöst im flüssigen Zustand vorliegen. Auch müssen die Zellen in der Matrix aus Biopolymer eine physiologische Umgebung vorfinden und eine gute Adhäsion zu dieser entwickeln, um sich in der Matrix aus Biopolymer möglichst gleichmäßig zu verteilen, dabei ihre biologische Aktivität behalten bzw. entwickeln sowie ihren Differenzierungsstatus behalten. Es besteht also Bedarf an einem Biopolymer, das den Eigenschaften der natürlichen extrazellulären Matrix sehr nahekommt.

Es ist deshalb die Aufgabe der vorliegenden Erfindung, ein Biopolymer bereitzustellen, das möglichst gut den zuvor genannten Anforderungen entspricht.

Hierfür stellt die vorliegende Erfindung ein Polypeptid gemäß dem Patentanspruch 1 oder 5, ein Polynucleotid nach Patentanspruch 7, das das erfindungsgemäße Polypeptid codiert, sowie eine Wirtszelle nach Patentanspruch 8, die das erfindungsgemäße Polypeptid exprimiert, bereit. Weiterhin wird auch ein Verfahren nach Patentanspruch 9 zur Herstellung einer Tinte für den 3D-Druck, wobei die Tinte ein Polypeptid enthält, sowie die Tinte nach Patentanspruch 10 bereitgestellt. Bereitgestellt werden auch Verfahren zur Herstellung eines 3D-Gerüsts nach den Patentansprüchen 11 und 13 sowie das nach diesen Verfahren erhältliche 3D-Gerüst nach den Patentansprüchen 15 und 16. Die Unteransprüche 2 bis 4, 6, 12 und 14 betreffen bevorzugte Ausführungsformen.

In einer ersten erfindungsgemäßen Ausführungsform betrifft die vorliegende Erfindung ein Polypeptid, das eine erste Aminosäuresequenz mit wenigstens 90 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 200 Aminosäuren aufweist. Die Verwendung der ersten Aminosäuresequenz ermöglicht die Bereitstellung eines Polypeptids, das in einer wässrigen Lösung bei Konzentrationen von 10 Gewichtsprozent pro Volumen (% w/v) bei Temperaturen von 18 °C und mehr, vorzugsweise von 16 °C und mehr, stärker bevorzugt von 14 °C und mehr, und am bevorzugtesten von 12 °C und mehr im flüssigen Zustand vorliegt, so dass das Polypeptid sich gut für den Einsatz in Biotinten für den 3D-Druck eignet. Besonders bevorzugt besteht das erfindungsgemäße Polypeptid aus der ersten Aminosäuresequenz und der jeweiligen weiteren Aminosäuresequenz an dem N-terminalen Ende und dem C-terminalen Ende der ersten Aminosäuresequenz. Die weitere Aminosäuresequenz an dem C-terminalen Ende der ersten Aminosäuresequenz kann gleich oder verschieden zu der weiteren Aminosäuresequenz an dem N-terminalen Ende der ersten Aminosäuresequenz sein.

Unter "Sequenzidentität" soll hierin verstanden werden, dass die Abfolge bzw. Reihenfolge der Aminosäuren in dem Polypeptid identisch ist. Unter "X % Sequenzidentität" soll demgemäß verstanden werden, dass die Abfolge bzw. Reigenfolge der Aminosäuresequenz zu X % identisch ist.

Unter "das Polypeptid an der ersten Aminosäuresequenz sowohl an deren N-terminalem Ende als auch an deren C-terminalem Ende jeweils eine weitere Aminosäuresequenz mit einer Länge von 0 bis Y Aminosäuren aufweist" soll hierin verstanden werden, dass die erste Aminosäuresequenz mit wenigstens X % Sequenzidentität mit SEQ ID Nr. 2 an beiden Enden über eine Peptidbindung mit einer weiteren (Peptid-)Sequenz mit 0 bis Y Aminosäuren Länge verbunden ist.

In einer Ausgestaltung der ersten Ausführungsform weist die erste Aminosäuresequenz des erfindungsgemäßen Polypeptids vorzugsweise wenigstens 92 %, stärker bevorzugt wenigstens 94 %, noch stärker bevorzugt wenigstens 95 %, noch stärker bevorzugt wenigstens 96 %, noch stärker bevorzugt wenigstens 97 %, noch stärker bevorzugt wenigstens 98 %, noch stärker bevorzugt wenigstens 99 % und am stärksten bevorzugt 100 % Sequenzidentität auf.

In einer Ausgestaltung der ersten Ausführungsform weist das Polypeptid an der ersten Aminosäuresequenz vorzugsweise sowohl an deren N-terminalem Ende als auch an deren C-terminalem Ende jeweils eine weitere Aminosäuresequenz mit einer Länge von 0 bis 150 Aminosäuren, stärker bevorzugt 0 bis 100 Aminosäuren, noch stärker bevorzugt 0 bis 80 Aminosäuren, noch stärker bevorzugt 0 bis 60 Aminosäuren, noch stärker bevorzugt 0 bis 50 Aminosäuren, noch stärker bevorzugt 0 bis 40 Aminosäuren, noch stärker bevorzugt 0 bis 30 Aminosäuren, noch stärker bevorzugt 0 bis 20 Aminosäuren und am stärksten bevorzugt 0 bis 10 Aminosäuren auf.

In einer zweiten erfindungsgemäßen Ausführungsform betrifft die vorliegende Erfindung ein Polypeptid, das wenigstens zwei Aminosäuresequenzen umfasst, die jeweils wenigstens 90 % Sequenzidentität mit SEQ ID Nr. 2 aufweisen. Die Erfinder der vorliegenden Anmeldung haben herausgefunden, dass auch die Wiederholung der genannten Aminosäuresequenz in dem erfindungsgemäßen Polypeptid zu einem Polypeptid führt, das sich aus den Gesichtspunkten des Gelierverhaltens und der Viskosität hervorragend für den Einsatz in Biotinten im 3D-Druck eignet.

In einer Ausgestaltung der zweiten Ausführungsform weist die jeweilige Aminosäuresequenz der wenigstens zwei Aminosäuresequenzen des erfindungsgemäßen Polypeptids vorzugsweise wenigstens 92 %, stärker bevorzugt wenigstens 94 %, noch stärker bevorzugt wenigstens 95 %, noch stärker bevorzugt wenigstens 96 %, noch stärker bevorzugt wenigstens 97 %, noch stärker bevorzugt wenigstens 98 %, noch stärker bevorzugt wenigstens 99 % und am stärksten bevorzugt 100 % Sequenzidentität auf.

In einer Ausgestaltung der zweiten Ausführungsform weist das Polypeptid vorzugsweise sowohl an dem N-terminalen Ende als auch an dem C-terminalen Ende der mindestens zwei Aminosäuresequenzen jeweils eine weitere Aminosäuresequenz mit einer Länge von 0 bis 100 Aminosäuren, stärker bevorzugt 0 bis 80 Aminosäuren, noch stärker bevorzugt 0 bis 60 Aminosäuren, noch stärker bevorzugt 0 bis 50 Aminosäuren, noch stärker bevorzugt 0 bis 40 Aminosäuren, noch stärker bevorzugt 0 bis 30 Aminosäuren, noch stärker bevorzugt 0 bis 20 Aminosäuren und am stärksten bevorzugt 0 bis 10 Aminosäuren auf.

In einer dritten erfindungsgemäßen Ausführungsform betrifft die vorliegende Erfindung ein Polynucleotid, das für das erfindungsgemäße Polypeptid nach einer der genannten Ausführungsformen codiert. Hierzu ist es bevorzugt, dass das Polynucleotid eine Nucleotidsequenz gemäß SEQ ID Nr. 5, SEQ ID Nr. 6, SEQ ID Nr. 7 oder SEQ ID Nr. 8 umfasst.

In einer vierten erfindungsgemäßen Ausführungsform betrifft die vorliegende Erfindung eine Wirtszelle, die das erfindungsgemäße Polypeptid nach einer der genannten Ausführungsformen exprimiert. Die Wirtszelle ist vorzugsweise eine, die frei von einem Enzymkomplex ist, das Prolin zu Hydroxyprolin umwandeln kann. Die Wirtszelle kann eine Säugetierzelle oder ein Mikroorganismus sein, beispielsweise Hefe, vorzugsweise Pichia Pastoris. Bei dem Enzymkomplex handelt es sich vorzugsweise um die Prolyl-4-hydroxylase. Es handelt sich bei dem erfindungsgemäßen Polypeptid folglich vorzugsweise um ein rekombinant hergestelltes Polypeptid.

In einer fünften erfindungsgemäßen Ausführungsform betrifft die vorliegende Erfindung ein Verfahren zur Herstellung einer Tinte für den 3D-Druck, wobei das Polypeptid in ein Lösungsmittel gegeben wird, wobei das Polypeptid eine erste Aminosäuresequenz mit wenigstens 85 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 300 Aminosäuren aufweist; oder wenigstens zwei Aminosäuresequenzen umfasst, die jeweils wenigstens 85 % Sequenzidentität mit SEQ ID Nr. 2 aufweisen, wobei das Polypeptid mit einer vernetzbaren Gruppe funktionalisiert sein kann. Besonders bevorzugt besteht das im erfindungsgemäßen Verfahren der fünften erfindungsgemäßen Ausführungsform eingesetzte Polypeptid aus der ersten Aminosäuresequenz und der jeweiligen weiteren Aminosäuresequenz an dem N-terminalen Ende und dem C-terminalen Ende der ersten Aminosäuresequenz. Die weitere Aminosäuresequenz an dem C-terminalen Ende der ersten Aminosäuresequenz kann gleich oder verschieden zu der weiteren Aminosäuresequenz an dem N-terminalen Ende der ersten Aminosäuresequenz sein.

Als Lösungsmittel kommen erfindungsgemäß Wasser oder wasserbasierte Puffersysteme aus der Zellkultur oder auch Zellkulturmedien infrage.

In einer Ausgestaltung der fünften Ausführungsform weist die erste Aminosäuresequenz des im erfindungsgemäßen Verfahren eingesetzten Polypeptids vorzugsweise wenigstens 87 %, stärker bevorzugt wenigstens 90 %, noch stärker bevorzugt wenigstens 92 %, noch stärker bevorzugt wenigstens 94 %, noch stärker bevorzugt wenigstens 95 %, noch stärker bevorzugt wenigstens 96 %, noch stärker bevorzugt wenigstens 97 %, noch stärker bevorzugt wenigstens 98 %, noch stärker bevorzugt wenigstens 99 % und am stärksten bevorzugt 100 % Sequenzidentität auf.

In einer Ausgestaltung der fünften Ausführungsform weist das im erfindungsgemäßen Verfahren eingesetzte Polypeptid an der ersten Aminosäuresequenz vorzugsweise sowohl an deren N-terminalem Ende als auch an deren C-terminalem Ende jeweils eine weitere Aminosäuresequenz mit einer Länge von 0 bis 250 Aminosäuren, stärker bevorzugt 0 bis 200 Aminosäuren, noch stärker bevorzugt 0 bis 150 Aminosäuren, noch stärker bevorzugt 0 bis 100 Aminosäuren, noch stärker bevorzugt 0 bis 80 Aminosäuren, noch stärker bevorzugt 0 bis 60 Aminosäuren, noch stärker bevorzugt 0 bis 50 Aminosäuren, noch stärker bevorzugt 0 bis 40 Aminosäuren, noch stärker bevorzugt 0 bis 30 Aminosäuren, noch stärker bevorzugt 0 bis 20 Aminosäuren und am stärksten bevorzugt 0 bis 10 Aminosäuren auf.

In einer sechsten Ausführungsform betrifft die vorliegende Erfindung eine Tinte für den 3D-Druck, die ein Polypeptid umfasst, das eine erste Aminosäuresequenz mit wenigstens 85 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 300 Aminosäuren aufweist; oder wenigstens zwei Aminosäuresequenzen umfasst, die jeweils wenigstens 85 % Sequenzidentität mit SEQ ID Nr. 2 aufweisen, wobei das Polypeptid mit einer vernetzbaren Gruppe funktionalisiert sein kann. Besonders bevorzugt besteht das in der erfindungsgemäßen Tinte eingesetzte Polypeptid aus der ersten Aminosäuresequenz und der jeweiligen weiteren Aminosäuresequenz an dem N-terminalen Ende und dem C-terminalen Ende der ersten Aminosäuresequenz. Die weitere Aminosäuresequenz an dem C-terminalen Ende der ersten Aminosäuresequenz kann gleich oder verschieden zu der weiteren Aminosäuresequenz an dem N-terminalen Ende der ersten Aminosäuresequenz sein.

In einer Ausgestaltung der sechsten Ausführungsform weist die erste Aminosäuresequenz des in der erfindungsgemäßen Tinte eingesetzten Polypeptids vorzugsweise wenigstens 87 %, stärker bevorzugt wenigstens 90 %, noch stärker bevorzugt wenigstens 92 %, noch stärker bevorzugt wenigstens 94 %, noch stärker bevorzugt wenigstens 95 %, noch stärker bevorzugt wenigstens 96 %, noch stärker bevorzugt wenigstens 97 %, noch stärker bevorzugt wenigstens 98 %, noch stärker bevorzugt wenigstens 99 % und am stärksten bevorzugt 100 % Sequenzidentität auf.

In einer Ausgestaltung der sechsten Ausführungsform weist das in der erfindungsgemäßen Tinte eingesetzte Polypeptid an der ersten Aminosäuresequenz vorzugsweise sowohl an deren N-terminalem Ende als auch an deren C-terminalem Ende jeweils eine weitere Aminosäuresequenz mit einer Länge von 0 bis 250 Aminosäuren, stärker bevorzugt 0 bis 200 Aminosäuren, noch stärker bevorzugt 0 bis 150 Aminosäuren, noch stärker bevorzugt 0 bis 100 Aminosäuren, noch stärker bevorzugt 0 bis 80 Aminosäuren, noch stärker bevorzugt 0 bis 60 Aminosäuren, noch stärker bevorzugt 0 bis 50 Aminosäuren, noch stärker bevorzugt 0 bis 40 Aminosäuren, noch stärker bevorzugt 0 bis 30 Aminosäuren, noch stärker bevorzugt 0 bis 20 Aminosäuren und am stärksten bevorzugt 0 bis 10 Aminosäuren auf.

In einer siebten erfindungsgemäßen Ausführungsform betrifft die vorliegende Erfindung ein Verfahren zur Herstellung eines 3D-Gerüsts unter Verwendung einer Tinte, vorzugsweise der erfindungsgemäßen Tinte der sechsten Ausführungsform, wobei die Tinte ein Polypeptid aufweist, wobei das Polypeptid eine erste Aminosäuresequenz mit wenigstens 80 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 350 Aminosäuren aufweist; oder wenigstens zwei Aminosäuresequenzen umfasst, die jeweils wenigstens 80 % Sequenzidentität mit SEQ ID Nr. 2 aufweisen, wobei das Polypeptid mit einer vernetzbaren Gruppe funktionalisiert (funktionalisiertes Polypeptid) sein kann, und wobei das Polypeptid mittels eines 3D-Druckverfahrens ausgehärtet wird.

In einer Ausgestaltung der siebten erfindungsgemäßen Ausführungsform ist das Polypeptid vorzugsweise mit einer vernetzbaren Gruppe funktionalisiert, und in dem 3D-Druckverfahren wird vorzugsweise lichtbasierte Formgebung verwendet.

Unter 3D-Druck oder 3D-Druckverfahren in der sechsten und siebten Ausführungsform versteht man sämtliche 3D-Druckverfahren, die im Stand der Technik bekannt sind, wie z.B. Extrusionsdruckverfahren, Tintenstrahldruckverfahren oder Druckverfahren unter lichtbasierter Formgebung, wie Stereolithographie oder volumetrischer/holographischer 3D-Druck, wobei Druckverfahren unter lichtbasierter Formgebung erfindungsgemäß bevorzugt sind.

Unter lichtbasierter Formgebung versteht man hierin vorzugsweise, dass das funktionalisierte Polypeptid aus der Tinte durch Lichteinstrahlung ausgehärtet wird, indem die vernetzbaren Gruppen miteinander verknüpft werden. Es wird also nur an den Stellen ausgehärtet, an denen auch mit ausreichend Lichtenergie bestrahlt wird (strukturierende Aushärtung). Dabei werden die Polypeptide untereinander vernetzt und es kommt zur Aushärtung des Polypeptids in dem Bereich, in dem ausreichend Lichtenergie eingestrahlt wird. Ein Belichtungsverfahren unter strukturierender Aushärtung des photovernetzbaren Polypeptids zur Bildung eines biokompatiblen vernetzten Polymers - wie es erfindungsgemäß eingesetzt wird - hat gegenüber anderen biologischen Druckverfahren wie dem Extrusionsverfahren, dem Tintenstrahlverfahren und dem Laser-gestützten Vorwärtstransfer-Verfahren den Vorteil einer überragenden Druckgeschwindigkeit, d.h. wieviel Volumen des zu druckenden Objekts pro Zeiteinheit aufgebaut werden kann. Einzigartig für die lichtbasierte strukturierte Aushärtung ist die Kombination aus hoher Druckgeschwindigkeit und hoher räumlicher Auflösung. Die Leistungsfähigkeit des Verfahrens beruht auf optischen Gesetzmäßigkeiten, und durch den Einsatz von handelsüblichen Optiken ist eine überlegende Auflösung bis in den Submikrometerbereich möglich.

Unter einem volumetrischen/holographischen 3D-Druckverfahren wird eine 3D-Struktur durch Lichteinstrahlung zeitgleich ausgehärtet, beispielsweise durch Lichtprojektion auf einen drehenden Zylinder, oder durch Kreuzung von mehreren Laserstrahlen, wodurch in den Punkten der Kreuzung der Laserstrahlen die Lichtintensität hoch genug für die lichtinduzierte Vernetzung ist.

Besonders bevorzugt wird als 3D-Druckverfahren ein stereolithographisches 3D-Druckverfahren verwendet. Unter einem stereolithographischen 3D-Druckverfahren versteht man eines, bei dem die Struktur des 3D-Gerüsts nach und nach durch punktuelles, schichtweises (in einer Schicht) oder volumetrisches/holographisches Aushärten hergestellt wird, wobei das Aushärten innerhalb einer Schicht erfindungsgemäß bevorzugt ist. Bei dem punktuellen Aushärten wird vorzugsweise ein Laser verwendet. Bei der schichtweisen Aushärtung wird vorzugsweise projektionsbasiert oder flächig bestrahlt, wobei vorzugsweise das sogenannte "digital light processing" verwendet wird. Aus Gründen der Geschwindigkeit ist ein projektionsbasiertes Belichtungsverfahren erfindungsgemäß bevorzugt.

Unter "photovernetzbar" soll hierin verstanden werden, dass das funktionalisierte Polypeptid durch Einwirkung von elektromagnetischer Strahlung und gegebenenfalls der Anwesenheit eines Photoinitiators vernetzt werden kann.

In einer achten erfindungsgemäßen Ausführungsform betrifft die vorliegende Erfindung ein Verfahren zur Herstellung eines 3D-Gerüsts unter Verwendung einer Tinte, vorzugsweise der erfindungsgemäßen Tinte der sechsten Ausführungsform, die ein Polypeptid aufweist, wobei das Polypeptid eine erste Aminosäuresequenz mit wenigstens 75 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 400 Aminosäuren aufweist; oder wenigstens zwei Aminosäuresequenzen umfasst, die jeweils wenigstens 75 % Sequenzidentität mit SEQ ID Nr. 2 aufweisen, wobei das Polypeptid mit einer vernetzbaren Gruppe funktionalisiert (funktionalisiertes Polypeptid) ist, und das Polypeptid mittels Stereolithographie ausgehärtet wird. Bezüglich der Stereolithographie gilt für die achte Ausführungsform auch alles, was in Bezug auf die Stereolithographie in Bezug auf die sechste und siebte Ausführungsform geschrieben wurde.

In den erfindungsgemäßen Verfahren der siebten und achten Ausführungsform ist es bevorzugt, dass aus dem Polypeptid ein 3D-Gerüst durch folgenden Schritt aufgebaut wird:
(i) Aushärten des funktionalisierten Polypeptids aus der Tinte durch Einstrahlen einer elektromagnetischen Strahlung (Belichtung) punktuell, in einer Schicht oder volumetrisch/holographisch, in dem Bereich der Tinte, in dem das Aushärten bzw. der Aufbau des 3D-Gerüsts erwünscht ist, wobei das Einstrahlen in einer Schicht erfindungsgemäß bevorzugt ist.

Durch die Belichtung wird aus dem funktionalisierten Polypeptid durch Vernetzung der funktionellen Gruppen ein vernetztes Polypeptid erhalten, das eine räumliche Struktur des 3D-Gerüsts vorgibt.

Unter dem Einstrahlen in einer Schicht soll hierin insbesondere verstanden werden, dass das aufzubauende, erfindungsgemäße 3D-Gerüst, während es sich in der Tinte befindet, flächig bestrahlt wird. Hierbei werden nur die Bereiche in der Fläche bestrahlt, bei denen räumlich eine Aushärtung erwünscht ist. Dabei können auch Bereiche der Fläche nicht bestrahlt werden, in denen keine Aushärtung erwünscht ist. In einem weiteren Schritt (i) kann dann aber auch in der gleichen Schicht des aufzubauenden, erfindungsgemäßen 3D-Gerüsts durch Einbringen der Schicht in eine weitere Tinte (gleich oder verschieden zur erfindungsgemäßen Tinte) und entsprechender Einstrahlung in den gewünschten Bereich der Schicht ein Bereich mit einem anderen Aufbau als im ersten Schritt (i) hergestellt werden. Weiterhin kann der Aufbau des erfindungsgemäßen 3D-Gerüsts aber auch dadurch erfolgen, dass auf die bereits ausgehärteten Bereiche durch Wiederholung des Schrittes (i) weitere ausgehärtete Bereiche gleicher oder unterschiedlicher Struktur und/oder Zusammensetzung aufgebracht werden. Auf diese Weise kann ein komplexes 3D-Gerüst mit unterschiedlichen Strukturen in jeder Schicht erhalten werden.

In einer Ausgestaltung des erfindungsgemäßen Verfahrens der siebten und achten Ausführungsformen ist es bevorzugt, dass der Aufbau des 3D-Gerüsts durch folgende Schritte erfolgt (vorzugsweise in der angegebenen Reihenfolge):
(I) Einbringen einer Tinte (der in der siebten oder achten Ausführungsform verwendeten Tinte) mit dem Polypeptid in ein Reaktionsgefäß,
(II) Eintauchen oder Vorhandensein einer Trägerplatte, auf der das 3D-Gerüst aufgebaut werden soll, in die Tinte,
(III) Einstrahlen einer elektromagnetischen Strahlung punktuell, in einer Schicht oder volumetrisch/holographisch in den Bereichen, in denen eine Aushärtung des Polypeptids gewünscht ist,
(IV) Erzeugen einer vernetzten Struktur des Polypeptids durch die elektromagnetische Strahlung,
(V) Eintauchen oder Vorhandensein der in Schritt (IV) erzeugten Struktur auf der Trägerplatte in eine weitere Tinte (entweder die in der siebten oder achten Ausführungsform verwendeten Tinte oder eine andere photovernetzbare oder photopolymerisierbare Tinte),
(VI) Einstrahlen einer elektromagnetischen Strahlung punktuell, in einer Schicht oder volumetrisch/holographisch in den Bereichen, in denen eine Aushärtung der weiteren Tinte gewünscht ist,
(VII) Erzeugen einer weiteren polymerisierten oder vernetzten Struktur durch die elektromagnetische Strahlung in Schritt (VI),
(VIII) Wiederholen der Schritte (V) bis (VII) mit jeweils einer Tinte (entweder die in der siebten oder achten Ausführungsform verwendeten Tinte oder eine andere photovernetzbare oder photopolymerisierbare Tinte), bis das 3D-Gerüst erzeugt ist.

Die in den wiederholten Schritten erzeugten Strukturen sind vorzugsweise durch kovalente Bindungen miteinander verbunden. Es sind jedoch auch nicht-kovalente Bindungen, beispielsweise auf der Grundlage physikalischer Wechselwirkungen, möglich. Das gesamte aufzubauende 3D-Gerüst kann auf einer Trägerplatte aufgebaut werden, die derart bewegt werden kann, dass das 3D-Gerüst nacheinander in gleiche oder verschiedene mit Tinte gefüllte Reaktionsgefäße bewegt wird und dort jeweils die lichtbasierte, strukturierende Aushärtung durchgeführt wird, so wie es weiter oben schon beschrieben ist. Sämtliche in der EP 3 018 531 B1 genannten Verfahren können beispielsweise erfindungsgemäß eingesetzt werden.

Durch die Wiederholung der Verfahrensschritte, in denen eine Vernetzung oder Polymerisierung der Tinten stattfindet, wird folglich ein schichtweiser, punktueller oder volumetrischer/holographischer Aufbau des 3D-Gerüsts erreicht. Dabei ist es möglich, ein 3D-Gerüst mit komplexer Struktur aufzubauen. Weiterhin können auch Hinterschnitte und überhängende Strukturen ausgebildet werden, da eine Vernetzung oder Polymerisierung der Tinte in einer bestimmten Schicht oder auch an einem bestimmten Punkt bei der punktuellen Einstrahlung auch dann erfolgen kann, wenn darunter kein bereits vernetztes oder polymerisiertes Material angeordnet ist, sondern lediglich noch nicht vernetzte oder nicht polymerisierte Flüssigkeit. Eine Polymerisierung oder Vernetzung von einer außerhalb der Schicht oder des Punktes vorliegenden Tinte/Polypeptid bleibt aus; vielmehr wird nur diejenige Tinte/Polypeptid vernetzt oder polymerisiert, die innerhalb der Schicht oder des Punktes liegt.

Folglich betrifft die vorliegende Erfindung in einer neunten erfindungsgemäßen Ausführungsform ein 3D-Gerüst, das nach einem Verfahren der siebten oder achten Ausführungsform erhältlich ist.

In einer weiteren Ausführungsform betrifft die vorliegende Erfindung ein erfindungsgemäßes 3D-Gerüst zur Verwendung in der Medizin bzw. die Verwendung des erfindungsgemäßen 3D-Gerüsts in der Medizin. Dabei ist es bevorzugt, dass das 3D-Gerüst als Matrix für lebende Zellen verwendet wird. Das 3D-Gerüst kann entweder nach dessen Herstellung mit lebenden Zellen besiedelt werden, oder die lebenden Zellen befinden sich in der Tinte, aus der das 3D-Gerüst hergestellt wird. Auf diese Weise können Gewebe oder In-vitro-Modelle aufgebaut werden, die dann in der Forschung verwendet oder auch implantiert werden können.

Die erfindungsgemäße Tinte der sechsten Ausführungsform oder die in den erfindungsgemäßen Verfahren der siebten und achten Ausführungsform eingesetzte Tinte kann folglich zusätzlich lebende Zellen aufweisen. Hierbei ist es auch möglich, dass unterschiedliche Tinten mit dem genannten Polypeptid verwendet werden, die sich aber bzgl. der verwendeten Zelltypen unterscheiden. Dabei können die verschiedenen verwendeten Zelltypen an unterschiedlichen, gewünschten Positionen innerhalb des 3D-Gerüsts sitzen.

Die verwendeten Tinten enthalten vorzugsweise jeweils einen oder mehrere biologische Zelltypen. Wenn es infolge der Einstrahlung der elektromagnetischen Strahlung zu einer Vernetzung kommt, werden die in der Flüssigkeit enthaltenen Zellen mit in das 3D-Gerüst eingebettet. Durch Verwendung von mehreren Tinten mit vorzugsweise einem oder mehreren unterschiedlichen biologischen Zelltypen kann ein komplexes 3D-Gerüst aufgebaut werden, das auch ein Blutgefäßstrukturnetzwerk aufweist. Die Verwendung einer Matrix aus dem vernetzten Polypeptid ermöglicht dabei den gerichteten Aufbau des erfindungsgemäßen 3D-Gerüsts.

Das erfindungsgemäße Polypeptid oder das erfindungsgemäß verwendete Polypeptid ist vorzugsweise mit einer vernetzbaren Gruppe funktionalisiert, die eine photoreaktive Gruppe ist.

In einer Variante ist die vernetzbare Gruppe eine Acrylgruppe, mittels derer die Vernetzung des erfindungsgemäßen Polypeptids oder des erfindungsgemäß verwendeten Polypeptids bewerkstelligt wird. Hierfür werden die primären Amine in den Seitenketten des erfindungsgemäßen Polypeptids oder des erfindungsgemäß verwendeten Polypeptids über eine nukleophile Substitutionsreaktion mit einem Anhydrid oder einem Säurechlorid umgesetzt, wobei Letzteres eine polymerisierbare Doppelbindung aufweist. Besonders bevorzugt wird hier ein Anhydrid oder Säurechlorid der Methacrylsäure oder des Carboxynorbornens eingesetzt.

Durch das Einbringen einer vernetzbaren Gruppe, bspw. einer Acrylgruppe, in das erfindungsgemäße Polypeptid oder das erfindungsgemäß verwendete Polypeptid wird dieses photovernetzbar gemacht. Durch die strahlungsinduzierte Kopplung der Acrylreste zwischen verschiedenen Molekülen des erfindungsgemäßen Polypeptids oder des erfindungsgemäß verwendeten Polypeptids wird eine vernetzte Matrix hergestellt.

Damit eine Photovernetzung über die vernetzbare Gruppe erreicht werden kann, wird vorzugsweise ein Radikalbildner (ein sogenannter Photoinitiator) eingesetzt, der bei einer ausgewählten Wellenlänge der im Rahmen des Verfahrens eingesetzten elektromagnetischen Strahlung Radikale bildet. Geeignete Radikalbildner sind beispielsweise Anthronderivate wie etwa Violanthron oder Isoviolanthron, Fluoreszein, Rubren, Anthrazenderivate, Tetrazenderivate, Benzanthron, Benzanthronil, Eosin, Levolinsäurederivate, Phosphin-Derivate, Mono- und Bis-Acyl-Phosphine, insbesondere Lithiumphenyl-2,4,6-trimethylbenzoylphosphinat, Metallocene, Acetophenone, Benzophenone, Xanthone, Quinone, Keton-Derivate, Hydroxyketone, Aminoketone, Benzoyl-Peroxide, Pyridin-Salze, Phenylglyoxylate und/oder lodonium-Salze.

Darüber hinaus kann der erfindungsgemäßen Tinte oder der erfindungsgemäß verwendeten Tinte eine Substanz zugesetzt werden, die eine Photovernetzung tieferer, flüssiger Schichten verhindert. So bleibt flüssige Lösung außerhalb der Fokusebene flüssig, auch wenn sie sich im Einstrahlbereich der über ihr liegenden Fokusebene befindet. Dies funktioniert durch eine Absorption der Substanz bei der Wellenlänge, bei der die Vernetzung stattfindet (vernetzende Wellenlänge). Das Abfangen findet in der Fokusebene statt, so dass kein Eindringen der vernetzenden Wellenlänge in tiefere Schichten möglich ist. Geeignet sind alle Substanzen, die in der gewünschten Wellenlänge absorbieren, wie etwa Farbstoffe.

Darüber hinaus ist es in einer Variante möglich, dass die erfindungsgemäße Tinte oder die erfindungsgemäß verwendete Tinte einen temperatursensitiven Gelbildner aufweist. Insbesondere ist der Einsatz eines invers-temperatursensitiven (auch als reverstemperatursensitiv bezeichneten) Gelbildners vorgesehen. Ein solcher Gelbildner wird mit steigender Temperatur fester. Durch ein Erwärmen des Reaktionsgefäßes erstarrt die Reaktionsflüssigkeit und bildet ein zunächst nur metastabiles Gel. Sollte die Tinte nicht gleichzeitig photovernetzt werden, kann durch ein nachfolgendes Abkühlen des 3D-Gerüsts das metastabile Gel wieder verflüssigt und abgepumpt werden. Bei gewöhnlichen temperatursensitiven Gelbildnern liegen die anzuwendenden Temperaturverhältnisse genau umgekehrt. So kann bei Bedarf beispielsweise eine Stützstruktur erstellt werden, so dass hängende Strukturen erzeugbar sind. Wird das metastabile Gel hingegen zumindest teilweise mit elektromagnetischer Strahlung in geeigneter Wellenlänge bestrahlt, kommt es zu einer Photovernetzung, so dass das metastabile Gel in diesen Bereichen in ein stabiles Gel bzw. Polymernetzwerk umgesetzt wird.

Mit anderen Worten ausgedrückt, kann durch den temperatursensitiven, insbesondere invers-temperatursensitiven, Gelbildner und eine Temperaturkontrolle des Reaktionsraumes noch einfacher mit hängenden Partien und Hinterschnitten oder Hohlräumen gearbeitet werden. Auch in dieser Variante kann weiterhin mit flüssigen Strukturen als Stütze gearbeitet werden.

Es ist darüber hinaus möglich, einen Temperaturgradienten vorzusehen, so dass ein metastabiles Gel nicht in allen Bereichen der mit dem temperatursensitiven, insbesondere invers-temperatursensitiven, Gelbildner versetzten Flüssigkeit erfolgt. Mit Hilfe eines solchen Gradienten können noch komplexere Strukturen erzeugt werden.

Alternativ oder zusätzlich zu der Bildung von Hohlräumen über die Verwendung eines Gelbildners können auch Enzyme zur Verdauung von Teilen des gebildeten 3D-Gerüsts eingesetzt werden. Das Prinzip ist das Folgende: Ein 3D-Gerüst mit Hohlräumen/Hinterschnitten (z.B. einem Kanalsystem) wird als solider Körper gedruckt, indem alle Hohlräume beim Druck mit einem Opfermaterial ausgefüllt sind, welches später (d.h. nach Abschluss des Drucks) durch Gabe des richtigen Enzyms aufgelöst werden kann. Das Opfermaterial ist bspw. ein verdaubares Polymer, das durch Zugabe eines verdauenden Enzyms verdaut wird. Dies ist eine elegante Strategie zum Schaffen von Hohlräumen mit stereolithografischen Druckverfahren. Beispielsweise kann eine Hyaluronidase (verdauendes Enzym) Hyaluronsäure (Opfermaterial) verdauen, so dass an der Stelle im 3D-Gerüst, in der Hyaluronsäure verdruckt wurde, durch Hyaluronidaseverdau ein Hohlraum entsteht.

Alternativ kann zur Schaffung eines Hohlraums/Hinterschnittes auch ein Photoblocker in der erfindungsgemäßen Tinte oder der erfindungsgemäß verwendeten Tinte eingesetzt werden, wobei der Photoblocker die Aushärtetiefe der Tinte einschränkt. Auf diese Weise kann auch die Aushärtung durch Einstrahlung in einer Schicht erfolgen, ohne dabei in der Einstrahltiefe alle Bereiche auszuhärten, die im Strahlengang liegen. Die Möglichkeit der schichtweisen Aushärtung erhöht die Druckgeschwindigkeit gegenüber punktuellen Verfahren immens.

In einer Variante wird zwischen einer Quelle für die elektromagnetische Strahlung (Strahlungsquelle), die zur Erzeugung der einen und/oder der weiteren elektromagnetischen Strahlung dient, und dem Reaktionsgefäß ein optisches System angeordnet, das zur Fokussierung der elektromagnetischen Strahlung auf die jeweilige Fokusebene in dem Reaktionsgefäß dient. Dabei ist es in einer Variante vorgesehen, dass eine Refokussierung dieses optischen Systems erfolgen kann, um die Schicht der Aushärtung innerhalb des Reaktionsgefäßes zu ändern. Eine derartige Refokussierung kann beispielsweise durch eine Veränderung des Abstands des optischen Systems zur Strahlungsquelle erreicht werden. Dabei kann ein computergesteuerter Schrittmotor vorgesehen sein, um eine entsprechende Bewegung des optischen Systems zu vermitteln. Beim optischen System kann es sich beispielsweise um ein System optischer Linsen oder - in einem konstruktiv besonders einfach gelagerten Fall - um eine einzelne Fokussierlinse handeln. Alternativ dazu kann aber auch der Punkt oder die Schicht der Aushärtung statisch sein und das aufzubauende 3D-Gerüst relativ zu der elektromagnetischen Strahlung bewegt werden.

Das jeweils gewählte Bestrahlungsmuster zur Herstellung des vernetzten 3D-Gerüsts kann beispielsweise von einem Computerprogramm bereitgestellt werden. So ist es denkbar, dass ein Anwender das herzustellende 3D-Gerüst mittels eines CAD-Programms erstellt. Das derart erstellte digitale Objekt wird dann von einem geeigneten Computerprogramm in einzelne Bestrahlungsebenen zerschnitten. Zu diesen Informationen werden Steuerinformationen für einen Drucker erstellt, mittels dessen das beschriebene Verfahren durchgeführt wird. Diese Steuerinformationen geben vor, wann welche photovernetzbare Tinte/Flüssigkeit in das Reaktionsgefäß eingebracht werden muss. Ferner geben diese Steuerinformationen vor, wann welches Bild einer Bestrahlungsebene auf die jeweilige Fokusebene im Reaktionsgefäß projiziert werden soll. Auf diese Weise lässt sich dann das zuvor am Computer erstellte 3D-Gerüst in ein reales 3D-Gerüst überführen.

In einer Variante weist die in den erfindungsgemäßen Verfahren verwendete elektromagnetische Strahlung eine Wellenlänge im Bereich von 200 nm bis 1000 nm (also eine Wellenlänge, die zwischen dem UV-Bereich und dem Infrarot-Bereich liegt), stärker bevorzugt im Bereich von 350 nm und 800 nm auf. Mit derartigen Wellenlängen lassen sich die bevorzugt als Radikalbildner eingesetzten Substanzen besonders gut anregen, so dass Radikale gebildet werden, um eine Vernetzung des die vernetzbaren Gruppen tragenden Polypeptids zu ermöglichen.

Weitere geeignete Wellenlängen der eingesetzten elektromagnetischen Strahlung liegen im Bereich von 250 nm bis 950 nm, insbesondere von 250 nm bis 850 nm, insbesondere von 300 nm bis 800 nm, insbesondere von 300 nm bis 750 nm, insbesondere von 300 nm bis 700 nm, insbesondere von 350 nm bis 650 nm und ganz besonders von 350 nm bis 410 nm.

Wie sich aus der vorherigen Darstellung der Herstellung des erfindungsgemäßen 3D-Gerüsts ergibt, kann der Schritt des 3D-Drucks des 3D-Gerüsts vollständig automatisiert durchgeführt werden, so dass ein Eingriff eines Benutzers nicht erforderlich ist. Dies erleichtert die Verfahrensanwendung zusätzlich.

Wenn das 3D-Gerüst auf einer Trägerplatte erzeugt wird, kann diese Trägerplatte nach Ende des Herstellungsprozesses komplett aus der noch verbliebenen Tinte im Reaktionsgefäß herausgehoben werden. Anschließend kann das erzeugte 3D-Gerüst vom Anwender von der Trägerplatte entfernt werden.

Weiterhin ist es bevorzugt, dass das erfindungsgemäße Polypeptid oder das erfindungsgemäß verwendete Polypeptid frei von der Aminosäure Hydroxyprolin ist. Auf diese Weise ist das Polypeptid eines, das in einer wässrigen Lösung bei Konzentrationen von bis zu 10 % (w/v) bei Temperaturen von 12 °C und mehr in flüssigem Zustand vorliegt.

Weiterhin kann das erfindungsgemäße Polypeptid oder das erfindungsgemäß verwendete Polypeptid auch eines mit einer Sequenz gemäß SEQ ID Nr. 1, SEQ ID Nr. 3 oder SEQ ID Nr. 4 sein.

Die vorliegende Erfindung soll nun anhand der folgenden Beispiele genauer erläutert werden:

### Abbildungen der Figuren:

- Fig. 1: zeigt temperaturabhängiges Gelierverhalten des erfindungsgemäßen Polypeptids (CoIMP) im Vergleich zu Gelatine;
- Fig. 2: zeigt ein ¹H-NMR des erfindungsgemäßen Polyeptids (ColMP) in unmodifizierter Form;
- Fig. 3: zeigt ein ¹H-NMR des erfindungsgemäßen Polpeptids (ColMP) modifiziert mit Methacryloylgruppen;
- Fig. 4: zeigt ein CD-Spektrum des erfindungsgemäßen (ColMP) Polypeptids bei 5 °C, 20 °C und 37 °C;
- Fig. 5: zeigt ein CD-Spektrum von Gelatine bei 5 °C, 20 °C und 37 °C.

### Aminosäureanalyse:

In ein Probenröhrchen aus Glas werden zu 2 bis 12 mg Polypeptid und 200 µL eines Gemisches aus konzentrierter Salzsäure und Trifluoressigsäure (Volumenverhältnis 2:1) gegeben. Das Probenröhrchen wird verschlossen und für 25 min bei 166°C hydrolysiert. Anschließend wird unter Vakuum vollständig getrocknet. Das Trockenprodukt wird in 1000 µL demineralisiertem Wasser gelöst. Die resultierende Lösung wird als Hydrolysat bezeichnet.

Für die Aminosäureanalytik wird ein Teil des Hydrolysats verdünnt, so dass die verdünnte Lösung einer Konzentration von 8 bis 18 µg des ausgehenden, nun hydrolysierten, Polypeptids entspricht (abhängig vom Instrument). Die Aminosäureanalyse wird mit einem Model 835 High-Speed Amino Acid Analyzer der Firma Hitachi unter Standardbedingungen für Proteinhydrolysate durchgeführt. Zur Auftrennung wird eine Kationenaustauschsäule verwendet. Die Detektion der Aminosäuren erfolgt mittels anschließender Verwendung einer Ninhydrin-Reaktion spektroskopisch. Das Absorptionsmaximum des gefärbten Hydroxyprolins liegt bei 440 nm. Ein Aminosäurestandard wurde verwendet, um das Instrument zu kalibrieren.

### Beispiel 1: Klonierungsarbeiten zur Herstellung eines Expressionskonstrukts für erfindungsgemäße rekombinante humane Polypeptide:

Das Gen zur rekombinanten Herstellung des erfindungsgemäßen Polypeptids (SEQ ID Nr. 2) ist eines, das einen Teil des humanen Kollagen I alpha 1 Proteins codiert. Die codierende DNA-Sequenz wurde für die Expression in Hefe (oder allgemeiner Organismus) codonoptimiert und als Produkt künstlicher Gensynthese (Invitrogen) bestellt. Die codierende Sequenz wurde anschließend mittels Restriktionsverdau und anschließender Ligation im Leserahmen hinter das α-Sekretionssignal des pPIC9K-Vektors (Thermo Fisher Scientific) kloniert. (SEQ ID Nr. 5) Die korrekte Integration des Zielgens wurde mittels DNA-Sequenzierung bestätigt.

Für die Aufreinigung mittels immobilisierter Metallionenchromatographie (IMAC) wurde in das oben beschriebene Konstrukt ein His-6-Tag eingefügt (SEQ ID Nr. 4). Dafür wurde durch PCR mit einem Primer, welche den His-6-Tag auf einem nicht bindenden Überhang codiert, die His-Tag codierende Sequenz zwischen die das Polypeptid codierende Sequenz und das Stopp-Codon eingefügt. Die korrekte Integration des His-6-Tags wurde durch DNA-Sequenzierung bestätigt. (SEQ ID Nr. 7.)

### Beispiel 2: Transformation des Expressionskonstrukts in Komagataella phaffii:

Für die Transformation wurde das zuvor generierte DNA-Konstrukt mittels Restriktionsverdau linearisiert und anschließend mittels Elektroporation in den GS115 *Komagataella phaffii* Stamm (Thermo Fisher Scientific) eingebracht. Positive Klone wurden durch Wachstum auf Selektionsmedium identifiziert.

### Beispiel 3: Expression des erfindungsgemäßen Polypeptids im Labormaßstab Bioreaktor:

Das rekombinante Polypeptid wird in einem Fed-Batch-Verfahren im 30 L-Maßstab hergestellt. Hierfür wird ein 30 L-Edelstahlbioreaktor (Sartorius) mit allen nötigen Sensoren (DO, pH, Temperatur) ausgestattet. Zusätzlich wird ein Methanolsensor (Raven Biotech) in den Reaktor verbaut. Die Fermentation wird durch Inokulation mit einer optischen Dichte bei 600 nm (OD₆₀₀) von 0,5 aus einer Vorkultur gestartet. Während der Fermentation werden Temperatur und pH konstant bei 30 °C bzw. 5 gehalten. Der Gelöstsauerstoff wird durch Variation der Rührerdrehzahl, Variation der Begasungsrate sowie Zugabe von Sauerstoff zur Zuluft bei Werten über 30 % gehalten.

Das Ende der Batch-Phase wird durch eine plötzliche Spitze im Messwert des Gelöstsauerstoffs erkannt, wenn die C-Quelle im Fermentationsmedium aufgebraucht ist. In der anschließenden Fed-Phase wird die Methanolkonzentration durch automatisierte Zugabe von Methanol basierend auf dem Messwert des Methanolsensors konstant bei 0,5 % gehalten. Die Fermentation wie oben beschrieben wird typischerweise nach 50-60 Stunden beendet.

### Beispiel 4: Aufreinigung des erfindungsgemäßen Polypeptids aus der Fermentationsbrühe:

Zur Aufreinigung werden zunächst die Zellen durch Zentrifugation entfernt. Grobe Verunreinigungen werden anschließend durch Filtration mit einem 1 µm-Filter abgetrennt, bevor kleinere Partikel und übrige Zellen durch Filtration durch eine 0,45 µm-Membran entfernt werden. Anschließend wird das Filtrat durch Tangentialflussfiltration mit einer 30 kDa-Membran gegen Reinstwasser dialysiert bis sich ein konstant bleibender Leitwert einstellt. Das Volumen wird anschließend durch Konzentration in dem Tangetialfiltrationsprozess reduziert und das Konzentrat durch Lyophilisation getrocknet.

Für die chromatographische Aufreinigung wird das trockene Produkt in geeignetem Laufpuffer gelöst und entweder mittels Kationenaustauschchromatographie oder IMAC weiter aufgereinigt. Das Eluat beider chromatographischer Verfahren wird anschließend mittels Tangetialflussfiltrration und einer 30 kDa-Membran gegen Reinstwasser dialysiert bis sich ein konstanter Leitwert einstellt, und anschließend durch Lyophilisation getrocknet.

### Beispiel 5: Biokompatibilitätstests:

Das aufgereinigte Polypeptid wird mittels MTT-Test auf seine Biokompatibilität untersucht. Dafür werden L929-Zellen in einer 96-Well-Platte für 24 Stunden nach Aussaat kultiviert. Anschließend wird das rekombinante Polypeptid in Konzentrationen von 1 % und 0,1 % (w/v) gelöst in Zellkulturmedium zu den Zellen hinzugegeben und die Zellen für weitere 24 Stunden kultiviert. Für die Analyse wird Thiazolylblau (MTT) in einer Konzentration von 1 mg/mL in Zellkulturmedium zu den Zellen gegeben und für 2 Stunden bei 37 °C inkubiert. Die Färbelösung wird anschließend entfernt und das gebildete Formazan in Isopropanol gelöst. Über die Absorptionsmessung der Isopropanollösung bei 570 nm und 650 nm wird die metabolische Aktivität der Zellen bestimmt. Die gemessenen Werte werden auf eine Kontrollgruppe ohne Substanzzugabe normalisiert. Zellen, die mit einer 1 %-igen Lösung des rekombinanten Materials behandelt wurden, zeigen normalerweise eine metabolische Aktivität von mindestens 70 % gegenüber der Kontrollgruppe.

### Beispiel 6: Funktionalisierung des aufgereinigten Produktes:

Zur Verwendung im 3D-Druck wird das aufgereinigte Polypeptid durch Anfügen von Methacryloylgruppen funktionalisiert. Hierfür wird das aufgereinigte Protein in einer Konzentration von 10 % (w/v) in 0,25 M CB-Puffer (pH 9) bei 25 °C gelöst. Anschließend wird der pH-Wert auf 9 eingestellt. Zum Start der Reaktion werden 0,1 mL Methacrylsäureanhydrid (MAA) (Sigma-Aldrich) pro Gramm trockenem Protein unter konstantem Rühren zu der Reaktion hinzugegeben. Ab der Zugabe von MAA wird unter Vermeidung direkter Lichteinstrahlung gearbeitet. Anschließend wird die Reaktion für 3 h unter Rückflusskühlung durchgeführt. Nach 3 h wird die Reaktion durch Verdünnen der Reaktionslösung mit CB-Puffer beendet. Die Reaktionslösung wird in einen Dialyseschlauch mit einem MWCO von 14 kDa überführt und für 3 Tage gegen H₂O dialysiert. Das Dialysewasser wird zweimal täglich gewechselt. Nach der Dialyse wird der Inhalt des Dialyseschlauches durch einen 0,45 µm-Filter filtriert und unter sterilen Bedingungen lyophilisiert. Ein Vergleichsmaterial (GelMA) wird analog aus Gelatine (Medella Pro, Gelita) hergestellt.

### Beispiel 7: Rheologische Analyse der Vernetzungseigenschaften:

Zur Analyse der Vernetzungseigenschaften wird das lyophilisierte und funktionalisierte Polypeptid in einer Konzentration von 10 % (w/v) in H₂O und einer finalen Konzentration von 0,1 % Lithium-Phenyl-2,4,6-trimethylbenzoylphosphinat (LAP) gelöst. Die Vernetzungseigenschaften durch Radikalkettenreaktion der eingefügten Methacryloylgruppen (Verlust und Speichermodul) vor, während und nach UV-Belichtung (365 nm, 19,95 mW/cm²) werden auf einem Rheometer (Anton-Paar) bei 1 Hz und 0,1 % Deformation bei 0,5 mm Messspalt und 37 °C gemessen. Zur Aushärtung während der Messung wird das Material für 180 s mit UV-Licht bestrahlt. GelMA wird als Vergleichsmaterial mit derselben Methode vermessen. Beide Materialien zeigen bei Bestrahlung mit UV-Licht eine rasche Zunahme des Speichermodules (G') als Folge der Polymerisationsreaktion.

### Beispiel 8: Bestimmung der Funktionalisierung mittels ¹H-NMR:

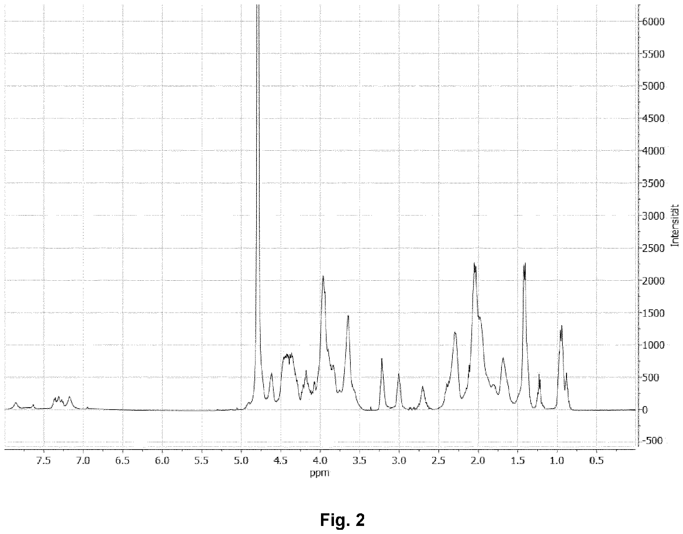

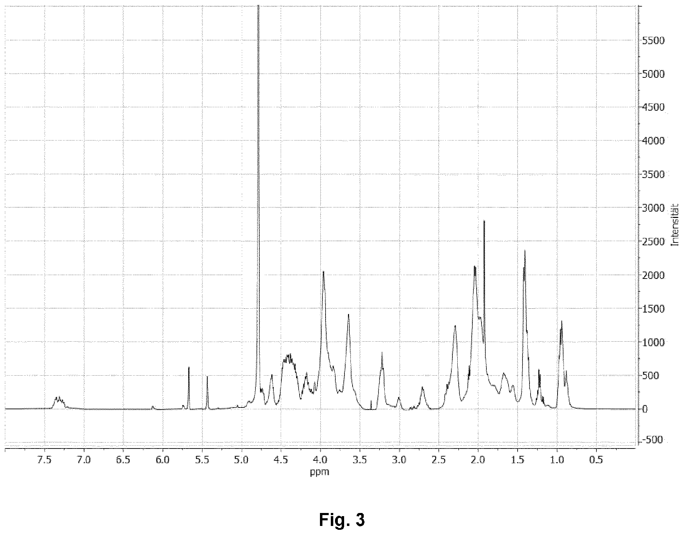

Zur Bestimmung des Funktionalisierungsgrades werden jeweils 20 mg unfunktionalisiertes und 20 mg funktionalisiertes Polypeptid (aus Beispiel 4 und 6) in 1 mL D₂O gelöst und mittels ¹H-NMR auf einem Ascend 400 (Bruker) bei 400 MHz vermessen. Der Funktionalisierungsgrad wird durch die Verhältnisse des Integrals des Lysinpeaks im unmodifizierten und modifizierten rekombinanten Material normalisiert auf den Phenylalaninpeak bestimmt. Das Spektrum des unmodifizierten Polypeptides (vgl. Fig. 2) zeigt im Gegensatz zu dem modifizierten Polypeptid (vgl. Fig. 3) einen höheren Lysinpeak bei ca. 2,9 ppm. Zusätzlich fehlen für das unmodifizierte Polypeptid die für die Methacryloylgruppe typischen Peaks bei ca. 5,3 ppm und ca. 5,6 ppm.

### Beispiel 9: Zelladhäsionstest mit erfindungsgemäßem quervernetztem funktionalisiertem Polypeptid:

Zum Test der Zelladhäsion wird das funktionalisierte Polypeptid in einer Konzentration von 10 % (w/v) in phosphatgepufferter Salzlösung (PBS) mit einer Konzentration von 0,1 % LAP gelöst. Die Biotinte wird in eine 96-Well-ultra-low-attachment (ULA)-Platte gegeben und durch Belichtung vollständig polymerisiert. Als Positivkontrolle wird eine 8 %-ige GelMA Tinte verwendet, als Negativkontrolle dient ein unbeschichtetes Well der Platte.

Für die Besiedelung werden primäre humane Fibroblasten aus der Vorhaut (FS-Fibroblasten) jeweils auf das Well mit polymerisiertem GelMA sowie quervernetztem, erfindungsgemäßem, funktionalisiertem Polypeptid (Beispiel 7) sowie der unbeschichteten Platte gegeben und für 24 Stunden bei 37 °C inkubiert.

Die Zellen werden anschließend per Lebend-Tot-Färbung mit Hoechst 33342, Calcein Red-Oragne AM und CellTox Green und Fluoreszenzmikroskopie untersucht.

FS-Fibroblasten auf dem rekombinantem Polypeptid zeigen einen geringeren Anteil an toten Zellen im Vergleich zu GelMA und darüber hinaus bessere Zelladhäsion mit gleichmäßigerer Verteilung. FS-Fibroblasten auf der unbehandelten Platte bilden Spheroide mit einem hohen Anteil toter Zellen.

### Beispiel 10: 3D-Biodruck des erfindungsgemäßen funktionalisierten Polypeptids (nach Beispiel 6):

Für den 3D-Biodruck wird das funktionalisierte Polypeptid wie zuvor beschrieben in 10 %-iger und 5 %-iger (w/v) Konzentration in PBS mit 0,1 % LAP gelöst. Anschließend wird die hergestellte Biotinte verwendet, um auf einem proprietären Multimaterialstereolithographen (Cellbricks) einen Zylinder mit 1 mm Höhe und 1 mm Radius zu drucken. Das Material zeigt dabei eine gute Druckbarkeit mit Polymerisation im bestrahlten Bereich innerhalb weniger Sekunden und klar definierten Konturen. Nach 24 h wurden FS-Fibroblasten (20.000 Zellen/Konstrukt) in dem Konstrukt wie bereits im Adhäsionstest beschrieben mittels Lebend-Tot-Färbung fluoreszenzmikroskopisch untersucht. FS-Fibroblasten zeigen Adhäsion an das Material des gedruckten Gerüsts über einen Zeitraum von mehreren Tagen mit Migration der Zellen durch das Material des Probenkörpers.

### Beispiel 11: Thermisches Gelierverhalten des erfindungsgemäßen, unfunktionalisierten Polypeptids (nach Beispiel 4):

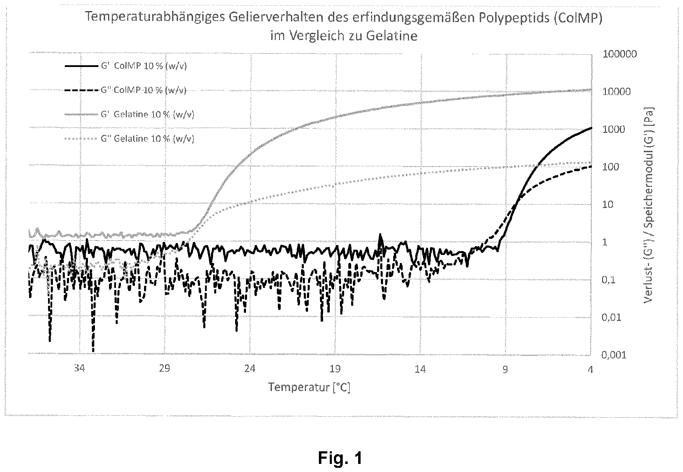

Da das erfindungsgemäße, funktionalisierte Polypeptid erfahrungsgemäß einen geringeren Gelierpunkt hat, wird zur Messung des thermischen Gelierverhaltens das erfindungsgemäße, unfunktionalisierte Polypeptid verwendet. Auf diese Weise kann sichergestellt werden, dass wenn das thermische Gelierverhalten des unfunktionalisierten Polypeptids geeignet ist, dass auch das funktionalisierte Polypeptid geeignet ist. Zur Messung des thermischen Gelierverhaltens wird das unfunktionalisierte Polypeptid in 10 % (w/v) in H₂O gelöst und Verlust und Speichermodul bei abnehmender Temperatur zwischen 37 °C und 4 °C mit einer Kühlrate von 0,5 °C/min gemessen. Als Vergleich wird eine 10 %-Gelatinelösung verwendet. Das rekombinante Peptid zeigt im Gegensatz zu Gelatine im ambienten Temperaturbereich kein thermisches Gelierverhalten (vgl. dazu Fig. 1). Dies kann durch einen Anstieg des Speichermoduls bei Reduzierung der Temperatur beobachtet werden. Während bei Gelatine ein Anstieg des Speichermoduls bereits unterhalb ca. 27 °C beobachtet wird, zeigt das rekombinante Polypeptid einen solchen Anstieg erst unterhalb ca. 12 °C und liegt damit bei gleicher Konzentration im Gegensatz zu Gelatine bei für den 3D-Biodruck typischen Arbeitstemperaturen (Raumtemperatur ca. 18-22 °C) in flüssiger Form vor.

### Beispiel 13: Messung des Circulardichroismus des erfindungsgemäßen, unfunktionalisierten Polypeptids (nach Beispiel 4):

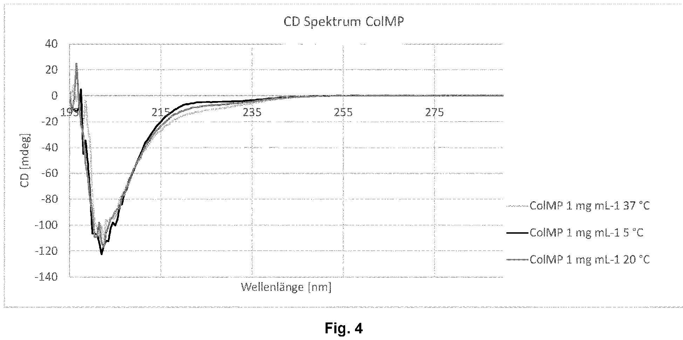

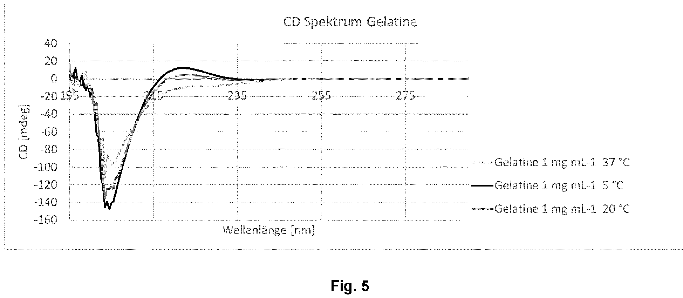

Zur Messung des Circulardichroismus wird das rekombinante Polypeptid bei einer Konzentration von 1 mg/mL in Reinstwasser gelöst und anschließend durch einen 0,22 µm Spritzenvorsatzfilter filtriert. Als Vergleichslösung wird Gelatine bei einer Konzentration von 1 mg/mL gelöst und durch einen 0,22 µm-Spritzenvorsatzfilter filtriert. Der Circulardichroismus der beiden Lösungen wird in einem J-815 CD-Spectrometer (Jasco) im Wellenlängenbereich von 190 bis 290 nm gemessen. Geplottet werden dabei die Mittelwerte von fünf aufeinanderfolgenden Messungen. Die Messungen wurden für beide Lösungen jeweils bei 5 °C, 20 °C und 37 °C durchgeführt (vgl. dafür Abb. 4 und Abb. 5.) Für Gelatine zeigt sich ein für Dreifachhelices typischer Peak bei ca. 220 nm bei 5 °C und 20 °C, jedoch nicht bei 37 °C. Für das rekombinante Polypeptid kann bei keinem der untersuchten Temperaturbereiche ein für Dreifachhelices typischer positiver Peak bei 220 nm beobachtet werden. Dies ist auf die fehlende oder geringere Prolylhydroxylierung des erfindungsgemäßen Polypeptids im Vergleich zum natürlich vorkommenden Polypeptidfragment zurückzuführen.

In den folgenden Tabellen sind die Sequenzlisten (SEQ ID Nr. 1 bis 8) beginnend mit dem N-terminalen Ende und endend mit dem C-terminalen Ende aufgelistet:

**Tabelle 1: Proteinsequenzen SEQ ID Nr. 1 und 2**

| | |
|---|---|
| SEQ ID Nr. 1 | MRFPSIFTAVLFAASSALAAPVNTTTEDETAQIPAEAVIGYSDLEGDFDVAV LPFSNSTNNGLLFINTTIASIAAKEEGVSLEKREAEAEFTGSPGSPGPDGKT GPPGPAGQDGRPGPPGPPGARGQAGPPGFPGPKGAAGEPGKAGERGV PGPPGAVGPAGKDGEAGAQGPPGPAGPAGERGEQGPAGSPGFQGLPG PAGPPGEAGKPGEQGVPGDLGAPGPSGARGERGFPGERGVQGPPGPA GPRGANGAPGNDGAKGDAGAPGAPGSQGAPGLQGMPGERGAAGLPGP KGDRGDAGPKGADGSPGKDGVRGLTGPIGPPGPAGAPGDKGESGPSGP AGPTGARGAPGDRGEPGPPGPAGFAGPPGADGQPGAKGEPGDAGAKG DAGPPGPAGPAGPPGPIGNVGAPGAKGARGSAGPPGATGFPGAAGRVG PPGPSGNAGPPGPPGPAGKEGGKGPRGETGPAGRPGEVGPPGPPGPA GEKGSPGADGPAGAPGTPGPQGIAGQRGVVGLPGQRGERGFPGLPGPS GEPGKQGPSGASGERGPPGPMGPPGLAGPPGESGREGAPGAEGSPGR DGSPGAKGDRGETGPAGPPGAPGAPGAPGPVGPAGKSGDRGETGPAG PTGPVGPVGARGPAGPQGPRGDKGETGEQGDRGIKGHRGFSGLQGPPG PPGSPGEQGPSGASGPAGPRGPPGSAGAPGKDGLNGLPGPIGPPGPRG RTGDAGPVGPPGPPGPPGPPGPP (1) EFTGSPGSPGPDGKTGPPGPAGQDGRPGPPGPPGARGQAGPPGFPGP KGAAGEPGKAGERGVPGPPGAVGPAGKDGEAGAQGPPGPAGPAGERG EQGPAGSPGFQGLPGPAGPPGEAGKPGEQGVPGDLGAPGPSGARGER GFPGERGVQGPPGPAGPRGANGAPGNDGAKGDAGAPGAPGSQGAPGL QGMPGERGAAGLPGPKGDRGDAGPKGADGSPGKDGVRGLTGPIGPPGP AGAPGDKGESGPSGPAGPTGARGAPGDRGEPGPPGPAGFAGPPGADG QPGAKGEPGDAGAKGDAGPPGPAGPAGPPGPIGNVGAPGAKGARGSAG PPGATGFPGAAGRVGPPGPSGNAGPPGPPGPAGKEGGKGPRGETGPA GRPGEVGPPGPPGPAGEKGSPGADGPAGAPGTPGPQGIAGQRGVVGLP GQRGERGFPGLPGPSGEPGKQGPSGASGERGPPGPMGPPGLAGPPGE SGREGAPGAEGSPGRDGSPGAKGDRGETGPAGPPGAPGAPGAPGPVG PAGKSGDRGETGPAGPTGPVGPVGARGPAGPQGPRGDKGETGEQGDR GIKGHRGFSGLQGPPGPPGSPGEQGPSGASGPAGPRGPPGSAGAPGKD GLNGLPGPIGPPGPRGRTGDAGPVGPPGPPGPPGPPGPP (2) |
| SEQ ID Nr. 2 | EFTGSPGSPGPDGKTGPPGPAGQDGRPGPPGPPGARGQAGPPGFPGP KGAAGEPGKAGERGVPGPPGAVGPAGKDGEAGAQGPPGPAGPAGERG EQGPAGSPGFQGLPGPAGPPGEAGKPGEQGVPGDLGAPGPSGARGER GFPGERGVQGPPGPAGPRGANGAPGNDGAKGDAGAPGAPGSQGAPGL QGMPGERGAAGLPGPKGDRGDAGPKGADGSPGKDGVRGLTGPIGPPGP AGAPGDKGESGPSGPAGPTGARGAPGDRGEPGPPGPAGFAGPPGADG QPGAKGEPGDAGAKGDAGPPGPAGPAGPPGPIGNVGAPGAKGARGSAG PPGATGFPGAAGRVGPPGPSGNAGPPGPPGPAGKEGGKGPRGETGPA GRPGEVGPPGPPGPAGEKGSPGADGPAGAPGTPGPQGIAGQRGVVGLP GQRGERGFPGLPGPSGEPGKQGPSGASGERGPPGPMGPPGLAGPPGE SGREGAPGAEGSPGRDGSPGAKGDRGETGPAGPPGAPGAPGAPGPVG PAGKSGDRGETGPAGPTGPVGPVGARGPAGPQGPRGDKGETGEQGDR GIKGHRGFSGLQGPPGPPGSPGEQGPSGASGPAGPRGPPGSAGAPGKD GLNGLPGPIGPPGPRGRTGDAGPVGPPGPPGPPGPPGPP (2) |

**Tabelle 2: Proteinsequenzen SEQ ID Nr. 3 und 4**

| | |
|---|---|
| SEQ ID Nr. 3 | MRFPSIFTAVLFAASSALAAPVNTTTEDETAQIPAEAVIGYSDLEGDFDVAVL PFSNSTNNGLLFINTTIASIAAKEEGVSLEKREAEAEFTGSPGSPGPDGKTG PPGPAGQDGRPGPPGPPGARGQAGPPGFPGPKGAAGEPGKAGERGVP GPPGAVGPAGKDGEAGAQGPPGPAGPAGERGEQGPAGSPGFQGLPGPA GPPGEAGKPGEQGVPGDLGAPGPSGARGERGFPGERGVQGPPGPAGP RGANGAPGNDGAKGDAGAPGAPGSQGAPGLQGMPGERGAAGLPGPKG DRGDAGPKGADGSPGKDGVRGLTGPIGPPGPAGAPGDKGESGPSGPAG PTGARGAPGDRGEPGPPGPAGFAGPPGADGQPGAKGEPGDAGAKGDA GPPGPAGPAGPPGPIGNVGAPGAKGARGSAGPPGATGFPGAAGRVGPP GPSGNAGPPGPPGPAGKEGGKGPRGETGPAGRPGEVGPPGPPGPAGEK GSPGADGPAGAPGTPGPQGIAGQRGVVGLPGQRGERGFPGLPGPSGEP GKQGPSGASGERGPPGPMGPPGLAGPPGESGREGAPGAEGSPGRDGS PGAKGDRGETGPAGPPGAPGAPGAPGPVGPAGKSGDRGETGPAGPTGP VGPVGARGPAGPQGPRGDKGETGEQGDRGIKGHRGFSGLQGPPGPPGS PGEQGPSGASGPAGPRGPPGSAGAPGKDGLNGLPGPIGPPGPRGRTGD AGPVGPPGPPGPPGPPGPPHHHHHH (3) |
| SEQ ID Nr. 4 | EFTGSPGSPGPDGKTGPPGPAGQDGRPGPPGPPGARGQAGPPGFPGPK GAAGEPGKAGERGVPGPPGAVGPAGKDGEAGAQGPPGPAGPAGERGE QGPAGSPGFQGLPGPAGPPGEAGKPGEQGVPGDLGAPGPSGARGERGF PGERGVQGPPGPAGPRGANGAPGNDGAKGDAGAPGAPGSQGAPGLQG MPGERGAAGLPGPKGDRGDAGPKGADGSPGKDGVRGLTGPIGPPGPAG APGDKGESGPSGPAGPTGARGAPGDRGEPGPPGPAGFAGPPGADGQPG AKGEPGDAGAKGDAGPPGPAGPAGPPGPIGNVGAPGAKGARGSAGPPG ATGFPGAAGRVGPPGPSGNAGPPGPPGPAGKEGGKGPRGETGPAGRPG EVGPPGPPGPAGEKGSPGADGPAGAPGTPGPQGIAGQRGVVGLPGQRG ERGFPGLPGPSGEPGKQGPSGASGERGPPGPMGPPGLAGPPGESGREG APGAEGSPGRDGSPGAKGDRGETGPAGPPGAPGAPGAPGPVGPAGKSG DRGETGPAGPTGPVGPVGARGPAGPQGPRGDKGETGEQGDRGIKGHRG FSGLQGPPGPPGSPGEQGPSGASGPAGPRGPPGSAGAPGKDGLNGLPG PIGPPGPRGRTGDAGPVGPPGPPGPPGPPGPPHHHHHH (4) |

**Tabelle 3: SEQ ID Nr. 5: Nucleotidsequenz codierend für Proteinsequenz SEQ ID Nr. 1**

| | |
|---|---|
| SEQ ID Nr. 5 | ATGAGATTTCCTTCAATTTTTACTGCAGTTTTATTCGCAGCATCCTCCGC ATTAGCTGCTCCAGTCAACACTACAACAGAAGATGAAACGGCACAAATT CCGGCTGAAGCTGTCATCGGTTACTCAGATTTAGAAGGGGATTTCGATG TTGCTGTTTTGCCATTTTCCAACAGCACAAATAACGGGTTATTGTTTATA AATACTACTATTGCCAGCATTGCTGCTAAAGAAGGGGTATCTCTCG AGAAAAGAGAGGCTGAAGCTGAATTCACTGGTTCCCCAGGTTCTCCAG GTCCAGATGGTAAAACTGGTCCACCAGGTCCTGCTGGACAAGATGGTA GACCAGGTCCACCTGGACCACCTGGTGCTAGAGGTCAAGCTGGTCCTC CTGGTTTTCCTGGTCCAAAAGGTGCAGCTGGTGAACCAGGTAAGGCTG GTGAAAGAGGTGTTCCAGGACCTCCAGGTGCTGTTGGACCTGCTGGTA AAGACGGTGAAGCTGGTGCTCAAGGTCCTCCAGGACCAGCCGGACCA GCAGGCGAGAGAGGTGAACAAGGACCTGCAGGATCTCCAGGATTTCAA GGTTTACCCGGTCCAGCTGGCCCACCAGGCGAAGCTGGTAAACCTGGT GAGCAAGGTGTTCCTGGTGATTTGGGTGCTCCTGGTCCTTCAGGTGCA AGAGGTGAAAGGGGTTTCCCAGGTGAACGTGGTGTACAGGGACCTCCT GGACCTGCCGGTCCTAGAGGTGCTAATGGTGCTCCAGGTAATGATGGT GCTAAGGGTGATGCCGGCGCTCCTGGTGCACCTGGATCTCAAGGTGC CCCAGGTCTACAAGGTATGCCCGGTGAGAGGGGTGCTGCTGGTTTGCC AGGACCTAAAGGTGATAGAGGTGATGCTGGACCAAAGGGTGCTGATGG ATCACCTGGTAAGGATGGTGTTAGAGGTTTGACTGGTCCAATCGGACC ACCAGGTCCAGCCGGTGCTCCCGGTGATAAGGGCGAATCTGGACCCT CTGGTCCTGCCGGACCAACTGGCGCTAGGGGTGCCCCTGGTGATCGT GGTGAGCCTGGACCTCCAGGTCCTGCCGGATTTGCTGGTCCACCTGGT GCAGATGGACAACCAGGTGCTAAAGGCGAACCAGGTGACGCCGGTGC CAAAGGTGACGCAGGCCCACCTGGTCCTGCAGGCCCCGCTGGACCAC CAGGACCTATTGGTAATGTTGGAGCCCCAGGTGCCAAGGGTGCCAGAG GATCAGCAGGCCCTCCAGGCGCTACTGGTTTCCCTGGCGCTGCTGGTA GAGTTGGCCCTCCTGGTCCATCTGGTAATGCTGGCCCTCCAGGACCTC CTGGTCCAGCCGGCAAAGAAGGTGGTAAAGGACCAAGGGGTGAAACT GGCCCAGCTGGTAGGCCAGGTGAGGTAGGACCACCTGGTCCTCCAGG TCCAGCTGGTGAAAAAGGTTCTCCTGGTGCTGACGGTCCCGCTGGCGC TCCCGGTACTCCCGGACCACAAGGTATTGCAGGTCAGAGAGGTGTTGT TGGATTGCCTGGACAAAGAGGTGAGAGAGGATTTCCTGGATTGCCAGG TCCATCAGGCGAACCTGGAAAGCAAGGACCTAGTGGTGCATCTGGCGA AAGAGGCCCACCAGGACCAATGGGTCCTCCTGGATTAGCAGGTCCTCC TGGCGAATCTGGTAGAGAAGGTGCTCCAGGCGCTGAAGGATCTCCTGG TAGAGATGGATCTCCCGGTGCAAAAGGCGATAGAGGCGAAACTGGTCC CGCTGGACCTCCTGGCGCTCCAGGTGCTCCTGGTGCTCCCGGTCCTGT CGGTCCTGCTGGAAAATCTGGTGACAGAGGTGAAACCGGACCTGCTGG CCCAACTGGTCCAGTCGGTCCAGTTGGAGCTAGAGGACCCGCAGGAC CTCAGGGTCCCAGAGGCGATAAGGGTGAGACTGGTGAACAGGGTGAC CGTGGTATTAAGGGTCACAGAGGTTTTTCTGGTCTGCAGGGCCCACCA GGTCCTCCAGGATCTCCAGGTGAACAAGGTCCTTCTGGTGCTTCTGGA CCAGCTGGCCCTCGTGGCCCACCTGGATCTGCTGGTGCTCCTGGAAAA GATGGTTTGAACGGTTTGCCTGGTCCTATAGGTCCTCCTGGTCCTAGAG GTAGAACTGGTGATGCCGGTCCTGTGGGACCTCCAGGTCCACCTGGTC CTCCAGGACCTCCTGGACCACCATAA (5) |

**Tabelle 4: SEQ ID Nr. 6: Nucleotidsequenz codierend für Proteinsequenz SEQ ID Nr. 2**

| | |
|---|---|
| SEQ ID Nr. 6 | GAATTCACTGGTTCCCCAGGTTCTCCAGGTCCAGATGGTAAAACTGGTC CACCAGGTCCTGCTGGACAAGATGGTAGACCAGGTCCACCTGGACCAC CTGGTGCTAGAGGTCAAGCTGGTCCTCCTGGTTTTCCTGGTCCAAAAG GTGCAGCTGGTGAACCAGGTAAGGCTGGTGAAAGAGGTGTTCCAGGAC CTCCAGGTGCTGTTGGACCTGCTGGTAAAGACGGTGAAGCTGGTGCTC AAGGTCCTCCAGGACCAGCCGGACCAGCAGGCGAGAGAGGTGAACAA GGACCTGCAGGATCTCCAGGATTTCAAGGTTTACCCGGTCCAGCTGGC CCACCAGGCGAAGCTGGTAAACCTGGTGAGCAAGGTGTTCCTGGTGAT TTGGGTGCTCCTGGTCCTTCAGGTGCAAGAGGTGAAAGGGGTTTCCCA GGTGAACGTGGTGTACAGGGACCTCCTGGACCTGCCGGTCCTAGAGG TGCTAATGGTGCTCCAGGTAATGATGGTGCTAAGGGTGATGCCGGCGC TCCTGGTGCACCTGGATCTCAAGGTGCCCCAGGTCTACAAGGTATGCC CGGTGAGAGGGGTGCTGCTGGTTTGCCAGGACCTAAAGGTGATAGAG GTGATGCTGGACCAAAGGGTGCTGATGGATCACCTGGTAAGGATGGTG TTAGAGGTTTGACTGGTCCAATCGGACCACCAGGTCCAGCCGGTGCTC CCGGTGATAAGGGCGAATCTGGACCCTCTGGTCCTGCCGGACCAACTG GCGCTAGGGGTGCCCCTGGTGATCGTGGTGAGCCTGGACCTCCAGGT CCTGCCGGATTTGCTGGTCCACCTGGTGCAGATGGACAACCAGGTGCT AAAGGCGAACCAGGTGACGCCGGTGCCAAAGGTGACGCAGGCCCACC TGGTCCTGCAGGCCCCGCTGGACCACCAGGACCTATTGGTAATGTTGG AGCCCCAGGTGCCAAGGGTGCCAGAGGATCAGCAGGCCCTCCAGGCG CTACTGGTTTCCCTGGCGCTGCTGGTAGAGTTGGCCCTCCTGGTCCAT CTGGTAATGCTGGCCCTCCAGGACCTCCTGGTCCAGCCGGCAAAGAAG GTGGTAAAGGACCAAGGGGTGAAACTGGCCCAGCTGGTAGGCCAGGT GAGGTAGGACCACCTGGTCCTCCAGGTCCAGCTGGTGAAAAAGGTTCT CCTGGTGCTGACGGTCCCGCTGGCGCTCCCGGTACTCCCGGACCACA AGGTATTGCAGGTCAGAGAGGTGTTGTTGGATTGCCTGGACAAAGAGG TGAGAGAGGATTTCCTGGATTGCCAGGTCCATCAGGCGAACCTGGAAA GCAAGGACCTAGTGGTGCATCTGGCGAAAGAGGCCCACCAGGACCAAT GGGTCCTCCTGGATTAGCAGGTCCTCCTGGCGAATCTGGTAGAGAAGG TGCTCCAGGCGCTGAAGGATCTCCTGGTAGAGATGGATCTCCCGGTGC AAAAGGCGATAGAGGCGAAACTGGTCCCGCTGGACCTCCTGGCGCTC CAGGTGCTCCTGGTGCTCCCGGTCCTGTCGGTCCTGCTGGAAAATCTG GTGACAGAGGTGAAACCGGACCTGCTGGCCCAACTGGTCCAGTCGGT CCAGTTGGAGCTAGAGGACCCGCAGGACCTCAGGGTCCCAGAGGCGA TAAGGGTGAGACTGGTGAACAGGGTGACCGTGGTATTAAGGGTCACAG AGGTTTTTCTGGTCTGCAGGGCCCACCAGGTCCTCCAGGATCTCCAGG TGAACAAGGTCCTTCTGGTGCTTCTGGACCAGCTGGCCCTCGTGGCCC ACCTGGATCTGCTGGTGCTCCTGGAAAAGATGGTTTGAACGGTTTGCCT GGTCCTATAGGTCCTCCTGGTCCTAGAGGTAGAACTGGTGATGCCGGT CCTGTGGGACCTCCAGGTCCACCTGGTCCTCCAGGACCTCCTGGACCA CCATAA (6) |

**Tabelle 5: SEQ ID Nr. 7: Nucleotidsequenz codierend für Proteinsequenz SEQ ID Nr. 3**

| | |
|---|---|
| SEQ ID Nr. 7 | ATGAGATTTCCTTCAATTTTTACTGCAGTTTTATTCGCAGCATCCTCCGCATTAGCTGCTCCA GTCAACACTACAACAGAAGATGAAACGGCACAAATTCCGGCTGAAGCTGTCATCGGTTAC TCAGATTTAGAAGGGGATTTCGATGTTGCTGTTTTGCCATTTTCCAACAGCACAAATAACG GGTTATTGTTTATAAATACTACTATTGCCAGCATTGCTGCTAAAGAAGAAGGGGTATCTCT CGAGAAAAGAGAGGCTGAAGCTGAATTCACTGGTTCCCCAGGTTCTCCAGGTCCAGATGG TAAAACTGGTCCACCAGGTCCTGCTGGACAAGATGGTAGACCAGGTCCACCTGGACCACC TGGTGCTAGAGGTCAAGCTGGTCCTCCTGGTTTTCCTGGTCCAAAAGGTGCAGCTGGTGA ACCAGGTAAGGCTGGTGAAAGAGGTGTTCCAGGACCTCCAGGTGCTGTTGGACCTGCTGG TAAAGACGGTGAAGCTGGTGCTCAAGGTCCTCCAGGACCAGCCGGACCAGCAGGCGAGA GAGGTGAACAAGGACCTGCAGGATCTCCAGGATTTCAAGGTTTACCCGGTCCAGCTGGCC CACCAGGCGAAGCTGGTAAACCTGGTGAGCAAGGTGTTCCTGGTGATTTGGGTGCTCCTG GTCCTTCAGGTGCAAGAGGTGAAAGGGGTTTCCCAGGTGAACGTGGTGTACAGGGACCT CCTGGACCTGCCGGTCCTAGAGGTGCTAATGGTGCTCCAGGTAATGATGGTGCTAAGGGT GATGCCGGCGCTCCTGGTGCACCTGGATCTCAAGGTGCCCCAGGTCTACAAGGTATGCCC GGTGAGAGGGGTGCTGCTGGTTTGCCAGGACCTAAAGGTGATAGAGGTGATGCTGGACC AAAGGGTGCTGATGGATCACCTGGTAAGGATGGTGTTAGAGGTTTGACTGGTCCAATCGG ACCACCAGGTCCAGCCGGTGCTCCCGGTGATAAGGGCGAATCTGGACCCTCTGGTCCTGC CGGACCAACTGGCGCTAGGGGTGCCCCTGGTGATCGTGGTGAGCCTGGACCTCCAGGTCC TGCCGGATTTGCTGGTCCACCTGGTGCAGATGGACAACCAGGTGCTAAAGGCGAACCAGG TGACGCCGGTGCCAAAGGTGACGCAGGCCCACCTGGTCCTGCAGGCCCCGCTGGACCACC AGGACCTATTGGTAATGTTGGAGCCCCAGGTGCCAAGGGTGCCAGAGGATCAGCAGGCC CTCCAGGCGCTACTGGTTTCCCTGGCGCTGCTGGTAGAGTTGGCCCTCCTGGTCCATCTGG TAATGCTGGCCCTCCAGGACCTCCTGGTCCAGCCGGCAAAGAAGGTGGTAAAGGACCAAG GGGTGAAACTGGCCCAGCTGGTAGGCCAGGTGAGGTAGGACCACCTGGTCCTCCAGGTC CAGCTGGTGAAAAAGGTTCTCCTGGTGCTGACGGTCCCGCTGGCGCTCCCGGTACTCCCG GACCACAAGGTATTGCAGGTCAGAGAGGTGTTGTTGGATTGCCTGGACAAAGAGGTGAG AGAGGATTTCCTGGATTGCCAGGTCCATCAGGCGAACCTGGAAAGCAAGGACCTAGTGGT GCATCTGGCGAAAGAGGCCCACCAGGACCAATGGGTCCTCCTGGATTAGCAGGTCCTCCT GGCGAATCTGGTAGAGAAGGTGCTCCAGGCGCTGAAGGATCTCCTGGTAGAGATGGATC TCCCGGTGCAAAAGGCGATAGAGGCGAAACTGGTCCCGCTGGACCTCCTGGCGCTCCAGG TGCTCCTGGTGCTCCCGGTCCTGTCGGTCCTGCTGGAAAATCTGGTGACAGAGGTGAAAC CGGACCTGCTGGCCCAACTGGTCCAGTCGGTCCAGTTGGAGCTAGAGGACCCGCAGGACC TCAGGGTCCCAGAGGCGATAAGGGTGAGACTGGTGAACAGGGTGACCGTGGTATTAAGG GTCACAGAGGTTTTTCTGGTCTGCAGGGCCCACCAGGTCCTCCAGGATCTCCAGGTGAACA AGGTCCTTCTGGTGCTTCTGGACCAGCTGGCCCTCGTGGCCCACCTGGATCTGCTGGTGCT CCTGGAAAAGATGGTTTGAACGGTTTGCCTGGTCCTATAGGTCCTCCTGGTCCTAGAGGTA GAACTGGTGATGCCGGTCCTGTGGGACCTCCAGGTCCACCTGGTCCTCCAGGACCTCCTG GACCACCACATCAcCATCATCATCACTAA (7) |

**Tabelle 6: SEQ ID Nr. 8: Nucleotidsequenz codierend für Proteinsequenz SEQ ID Nr. 4**

| | |
|---|---|
| SEQ ID Nr. 8 | GAATTCACTGGTTCCCCAGGTTCTCCAGGTCCAGATGGTAAAACTGGTCCACCAGGTCCTG CTGGACAAGATGGTAGACCAGGTCCACCTGGACCACCTGGTGCTAGAGGTCAAGCTGGTC CTCCTGGTTTTCCTGGTCCAAAAGGTGCAGCTGGTGAACCAGGTAAGGCTGGTGAAAGAG GTGTTCCAGGACCTCCAGGTGCTGTTGGACCTGCTGGTAAAGACGGTGAAGCTGGTGCTC AAGGTCCTCCAGGACCAGCCGGACCAGCAGGCGAGAGAGGTGAACAAGGACCTGCAGG ATCTCCAGGATTTCAAGGTTTACCCGGTCCAGCTGGCCCACCAGGCGAAGCTGGTAAACCT GGTGAGCAAGGTGTTCCTGGTGATTTGGGTGCTCCTGGTCCTTCAGGTGCAAGAGGTGAA AGGGGTTTCCCAGGTGAACGTGGTGTACAGGGACCTCCTGGACCTGCCGGTCCTAGAGGT GCTAATGGTGCTCCAGGTAATGATGGTGCTAAGGGTGATGCCGGCGCTCCTGGTGCACCT GGATCTCAAGGTGCCCCAGGTCTACAAGGTATGCCCGGTGAGAGGGGTGCTGCTGGTTTG CCAGGACCTAAAGGTGATAGAGGTGATGCTGGACCAAAGGGTGCTGATGGATCACCTGG TAAGGATGGTGTTAGAGGTTTGACTGGTCCAATCGGACCACCAGGTCCAGCCGGTGCTCC CGGTGATAAGGGCGAATCTGGACCCTCTGGTCCTGCCGGACCAACTGGCGCTAGGGGTGC CCCTGGTGATCGTGGTGAGCCTGGACCTCCAGGTCCTGCCGGATTTGCTGGTCCACCTGGT GCAGATGGACAACCAGGTGCTAAAGGCGAACCAGGTGACGCCGGTGCCAAAGGTGACGC AGGCCCACCTGGTCCTGCAGGCCCCGCTGGACCACCAGGACCTATTGGTAATGTTGGAGC CCCAGGTGCCAAGGGTGCCAGAGGATCAGCAGGCCCTCCAGGCGCTACTGGTTTCCCTGG CGCTGCTGGTAGAGTTGGCCCTCCTGGTCCATCTGGTAATGCTGGCCCTCCAGGACCTCCT GGTCCAGCCGGCAAAGAAGGTGGTAAAGGACCAAGGGGTGAAACTGGCCCAGCTGGTA GGCCAGGTGAGGTAGGACCACCTGGTCCTCCAGGTCCAGCTGGTGAAAAAGGTTCTCCTG GTGCTGACGGTCCCGCTGGCGCTCCCGGTACTCCCGGACCACAAGGTATTGCAGGTCAGA GAGGTGTTGTTGGATTGCCTGGACAAAGAGGTGAGAGAGGATTTCCTGGATTGCCAGGTC CATCAGGCGAACCTGGAAAGCAAGGACCTAGTGGTGCATCTGGCGAAAGAGGCCCACCA GGACCAATGGGTCCTCCTGGATTAGCAGGTCCTCCTGGCGAATCTGGTAGAGAAGGTGCT CCAGGCGCTGAAGGATCTCCTGGTAGAGATGGATCTCCCGGTGCAAAAGGCGATAGAGG CGAAACTGGTCCCGCTGGACCTCCTGGCGCTCCAGGTGCTCCTGGTGCTCCCGGTCCTGTC GGTCCTGCTGGAAAATCTGGTGACAGAGGTGAAACCGGACCTGCTGGCCCAACTGGTCCA GTCGGTCCAGTTGGAGCTAGAGGACCCGCAGGACCTCAGGGTCCCAGAGGCGATAAGGG TGAGACTGGTGAACAGGGTGACCGTGGTATTAAGGGTCACAGAGGTTTTTCTGGTCTGCA GGGCCCACCAGGTCCTCCAGGATCTCCAGGTGAACAAGGTCCTTCTGGTGCTTCTGGACCA GCTGGCCCTCGTGGCCCACCTGGATCTGCTGGTGCTCCTGGAAAAGATGGTTTGAACGGT TTGCCTGGTCCTATAGGTCCTCCTGGTCCTAGAGGTAGAACTGGTGATGCCGGTCCTGTGG GACCTCCAGGTCCACCTGGTCCTCCAGGACCTCCTGGACCACCACATCACCATCATCATCAC TAA (8) |

## Patentansprüche

1. Polypeptid umfassend eine erste Aminosäuresequenz mit wenigstens 90 % Sequenzidentität mit SEQ ID Nr. 2, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 200 Aminosäuren aufweist.

2. Polypeptid nach Anspruch 1, wobei die erste Aminosäuresequenz eine Sequenzidentität mit SEQ ID Nr. 2 von wenigstens 95 % aufweist.

3. Polypeptid nach Anspruch 1 oder 2, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 150 Aminosäuren aufweist.

4. Polypeptid nach einem der Ansprüche 1 bis 3, das im Wesentlichen frei von der Aminosäure Hydroxyprolin ist.

5. Polypeptid umfassend wenigstens zwei Aminosäuresequenzen, die jeweils wenigstens 90 % Sequenzidentität mit SEQ ID Nr. 2 aufweisen.

6. Polypeptid gemäß einem der Ansprüche 1 bis 5, das mit mindestens einer vernetzbaren Gruppe funktionalisiert ist, wobei die vernetzbare Gruppe eine photovernetzbare Gruppe sein kann.

7. Polynucleotid codierend für das Polypeptid nach einem der Ansprüche 1 bis 5.

8. Wirtszelle exprimierend das Polypeptid nach einem der Ansprüche 1 bis 5.

9. Verfahren zur Herstellung einer Tinte für den 3D-Druck, wobei das Polypeptid in ein Lösungsmittel gegeben wird, wobei das Polypeptid
(a) eine erste Aminosäuresequenz mit wenigstens 85 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 300 Aminosäuren aufweist;
oder
(b) wenigstens zwei Aminosäuresequenzen umfasst, die jeweils wenigstens 85 % Sequenzidentität mit SEQ ID Nr. 2 aufweisen,
wobei das Polypeptid mit mindestens einer vernetzbaren Gruppe funktionalisiert sein kann, wobei die vernetzbare Gruppe eine photovernetzbare Gruppe sein kann.

10. Tinte für den 3D-Druck, die ein Polypeptid umfasst, das
(a) eine erste Aminosäuresequenz mit wenigstens 85 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 300 Aminosäuren aufweist;
oder
(b) wenigstens zwei Aminosäuresequenzen umfasst, die jeweils wenigstens 85 % Sequenzidentität mit SEQ ID Nr. 2 aufweisen,
wobei das Polypeptid mit mindestens einer vernetzbaren Gruppe funktionalisiert sein kann, wobei die vernetzbare Gruppe eine photoreaktive Gruppe sein kann.

11. Verfahren zur Herstellung eines 3D-Gerüsts unter Verwendung einer Tinte, die ein Polypeptid aufweist, wobei das Polypeptid
(a) eine erste Aminosäuresequenz mit wenigstens 80 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 350 Aminosäuren aufweist;
oder
(b) wenigstens zwei Aminosäuresequenzen umfasst, die jeweils wenigstens 80 % Sequenzidentität mit SEQ ID Nr. 2 aufweisen,
wobei das Polypeptid mit einer vernetzbaren Gruppe funktionalisiert sein kann, und wobei das Polypeptid mittels eines 3D-Druckverfahrens, vorzugsweise durch eine lichtinduzierte chemische Reaktion, ausgehärtet wird.

12. Verfahren nach Anspruch 11, wobei das Polypeptid mit mindestens einer vernetzbaren Gruppe funktionalisiert ist, wobei die vernetzbare Gruppe eine photoreaktive Gruppe sein kann, und in dem 3D-Druckverfahren lichtbasierte Formgebung verwendet wird.

13. Verfahren zur Herstellung eines 3D-Gerüsts unter Verwendung einer Tinte, die ein Polypeptid aufweist, wobei das Polypeptid
(a) eine erste Aminosäuresequenz mit wenigstens 75 % Sequenzidentität mit SEQ ID Nr. 2 umfasst, wobei das Polypeptid an der ersten Aminosäuresequenz sowohl an deren N-terminalem Ende als auch an deren C-terminalem Ende jeweils eine weitere Aminosäuresequenz mit einer Länge von 0 bis 400 Aminosäuren aufweist;
oder
(b) wenigstens zwei Aminosäuresequenzen umfasst, die jeweils wenigstens 75 % Sequenzidentität mit SEQ ID Nr. 2 aufweisen,
wobei das Polypeptid mit mindestens einer vernetzbaren Gruppe funktionalisiert ist, wobei die vernetzbare Gruppe eine photoreaktive Gruppe sein kann, und das Polypeptid mittels Stereolithographie ausgehärtet wird.

14. Verfahren nach einem der Ansprüche 1 bis 13, wobei die Tinte zusätzlich lebende Zellen aufweist.

15. 3D-Gerüst, erhältlich nach einem Verfahren gemäß einem der Ansprüche 11 bis 14.

16. 3D-Gerüst nach Anspruch 15 zur Verwendung in der Medizin.
